# EUROPEAN PATENT APPLICATION

(11) **EP 4 685 805 A1**
(43) Date of publication of application: **28.01.2026**
(21) Application number: 24190350.9
(22) Date of filing: 23.07.2024
(51) Int. Cl.: G16H 10/20, G16H 50/20

(54) **EVALUATING USER TRUST IN ARTIFICIAL INTELLIGENCE-BASED CLINICAL DECISION SUPPORT SYSTEMS**

(71) Applicant: Koninklijke Philips N.V., 5656 AG Eindhoven (NL)
(72) Inventor: BUIL, Vincentius Paulus, Eindhoven (NL); WIJN, Victor, Eindhoven (NL); HENDRIKS, Monique, 5656AG Eindhoven (NL); KUHLMANN, Daan, Eindhoven (NL); PLUYTER, Jon Ragnar, Eindhoven (NL); BAKKER, Saskia, Eindhoven (NL)
(74) Representative: Philips Intellectual Property & Standards

(57) **Abstract**

Techniques for evaluating user interactions with a clinical decision support (CDS) system are disclosed. User interaction data is received associated with a user response to an advice item generated by the CDS system. The user interaction data is evaluated, such as to determine a user trust level associated with the advice item and/or the CDS system. A corrective action is generated based on the evaluation of the user interaction data. In some examples, the disclosed techniques identify and correct for user under-trust or over-trust in artificial intelligence (AI)-based CDS systems.

## Description

### FIELD OF THE INVENTION

This specification relates to evaluation of artificial intelligence-based clinical decision support and user interactions therewith. Various embodiments can evaluate user trust in one or more outputs of a clinical decision support system and take one or more actions responsive to over-trust or under-trust in the one or more outputs.

### BACKGROUND OF THE INVENTION

Clinical decision support (CDS) provides physicians, technicians, staff, and subjects (e.g., patients) with information to assist with delivery of healthcare. CDS can refer to a variety of tools to enhance decision making in a clinical workflow. For example, CDS can include technologies that provide alerts and reminders to care providers and subjects, clinical guidelines, condition-specific order sets, focused patient data reports and summaries, documentation templates, diagnostic support, contextually relevant reference information, and the like. CDS systems may comprise or use various artificial intelligence (AI) and/or machine learning (ML) technologies. CDS systems can be used, for example, in relation to various imaging technologies, such as ultrasound, magnetic resonance imaging (MRI), computed tomography (CT), and so forth. Additionally or alternatively, CDS systems can be used in relation to various other medical technologies or processes, such as patient monitoring and surgery planning. Example uses of CDS include radiology and ultrasound (e.g., for automatic segmentation or localization, cancer identification, organ recognition, automatic measurements, automatic reporting), patient monitoring (e.g., hemo stability index, acerbation prediction, septic shock recognition, etc.), and surgery/surgery planning (e.g., tumor resectability, instrument routing, robot planning and control).

### SUMMARY OF THE INVENTION

Apparatuses, systems, and methods for evaluating user interactions with an Al-based CDS system are disclosed. The disclosed technology can be used, for example, to evaluate user trust in a CDS system and/or in one or more outputs of the CDS system based on user interactions with the CDS system. The disclosed technology takes one or more actions to encourage appropriate user trust in the CDS system, such as to correct for over-trust or under-trust in the CDS system.

In accordance with at least one example disclosed herein, a computer-implemented method is disclosed for evaluating user interactions with a CDS system. An output of the CDS system is received based on data acquired via a medical device. In some implementations, the method is applied to other decision support systems and/or computing devices. User interaction data associated with the output of the CDS system is accessed, the user interaction data comprising at least one action responsive to the output of the CDS system, the at least one action indicating user agreement or disagreement with the output of the CDS system. The at least one action indicating user agreement or disagreement comprises a user input provided via a user interface of the CDS system. The user interaction data is evaluated based at least in part on an accuracy of the CDS system. A corrective action is generated based on the evaluating of the user interaction data, and the corrective action is provided via the user interface. The corrective action can comprise modification of a visual characteristic, or display of a prompt or notification via the user interface. In some implementations, the corrective action includes an auditory component and/or a tactile component.

In some implementations, a confidence score associated with the output of the CDS system is determined, and the user interaction is evaluated based at least in part on the confidence score.

In various embodiments, evaluating the user interaction data based at least in part on the accuracy of the CDS system includes determining a user trust level associated with the output of the CDS system, a user trust level associated with the CDS system, or both. The user trust level associated with the CDS system can be based on at least one user characteristic, which can be a user profile or a history of user interactions with the CDS system or a different CDS system.

In some implementations, the user trust level is compared to a threshold value based on an appropriate trust level corresponding to the accuracy of the CDS system, and the corrective action is based on the comparing of the user trust level to the threshold value.

In some implementations, the user interaction data is evaluated using an artificial intelligence (AI) model trained using a training dataset comprising different user interaction data, corresponding user trust data, and corresponding guardrail corrections.

In some implementations, the user interaction data comprises sensor data associated with a user. The sensor data can comprise user images, and evaluating the user interaction data can comprise applying computer vision, gaze detection, or both.

In some implementations, evaluating the user interaction data comprises detecting user attentiveness or fatigue.

In some implementations, an effectiveness of the corrective action is determined based at least in part on a user input responsive to the corrective action or different user interaction data of a different user interaction.

In accordance with other examples disclosed herein, a computing system is disclosed comprising at least one processor and at least one memory carrying instructions configured to cause the computing system to perform one or more methods disclosed herein.

In accordance with other examples, a non-transitory computer-readable medium is disclosed carrying instructions that, when executed by a processor or a computing system, cause performance of one or more methods disclosed herein.

### BRIEF DESCRIPTION OF THE DRAWINGS

Fig. 1 is a block diagram illustrating a computing device implementing a system for evaluating user interactions with a CDS system arranged in accordance with principles of the present disclosure.
Fig. 2 is a block diagram illustrating a workflow for evaluating user interactions with an AI-based CDS system in accordance with principles of the present disclosure.
Figs. 3A-3D are display diagrams illustrating outputs of a system for evaluating user interactions with a CDS system arranged in accordance with principles of the present disclosure.
Fig. 4 is a sequence diagram illustrating a process for evaluating user interactions with a CDS system in accordance with principles of the present disclosure.

### DETAILED DESCRIPTION OF THE EMBODIMENTS

The following description of certain examples is merely illustrative in nature and is in no way intended to limit the disclosed technology or its applications or uses. In the following detailed description of examples of the present apparatuses, systems, and methods, reference is made to the accompanying drawings which form a part hereof, and in which are shown by way of illustration specific examples in which the described apparatuses, systems and methods can be practiced. These examples are described in sufficient detail to enable those skilled in the art to practice the presently disclosed apparatuses, systems, and methods, and it is to be understood that other examples may be utilized and that structural and logical changes may be made without departing from the spirit and scope of the present disclosure. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present technology is defined only by the appended claims.

Healthcare providers face difficult technical challenges related to use of CDS systems, such as AI-based CDS systems (e.g., CDS systems that include or use one or more AI or ML models). While CDS systems can provide valuable assistance in various clinical workflows, such systems cannot achieve perfect accuracy. Accordingly, users of CDS systems should not "over-trust" CDS systems - that is, users should critically evaluate CDS systems and outputs thereof based on users' knowledge and training to avoid relying on outputs of the CDS system that are inaccurate or erroneous. On the other hand, users may "under-trust" CDS systems by rejecting outputs that are correct. For example, studies have shown that under-trust in CDS systems may cause radiologists to overlook or disregard small cancers that were accurately identified by AI-based CDS systems. Appropriate trust - not too much and not too little - improves collaboration between users and AI-based CDS systems and improves delivery of healthcare services to subjects, such as by improving reliability of diagnoses and supporting better decision making by physicians or technicians.

Moreover, existing systems may be unable to evaluate user trust in a CDS system in real time (e.g., in seconds or less). For example, users of CDS systems may rely on surveys or questionnaires, which may be completed manually, to assess user trust and encourage appropriate trust in CDS systems. Such systems may be ineffective because they are unable to encourage appropriate trust while a user is using a CDS system. Moreover, such systems may be ineffective because they may rely on users to self-report their trust levels, rather than directly evaluating user trust levels based on interactions with the CDS.

As used herein, "over-trust" and "under-trust" may relate to a user's acceptance of outputs of a CDS system relative to the accuracy of the CDS system and/or outputs thereof. For example, a CDS system may be associated with an accuracy level (e.g., a range), indicating that outputs of the CDS system are accurate a certain percentage of the time (e.g., 50%, 60%, 70%, 80%, 90%). In some applications, accuracy may include or be associated with a confidence score. Accuracy levels can apply to all or substantially all outputs of a CDS system, or accuracy levels can apply to specific outputs or types of outputs of a CDS system. For example, a given CDS system may have a greater accuracy level in identifying a first type of tumor in a medical image, as compared to an accuracy level in identifying a second type of tumor in a medical image. Over-trust can refer to a situation where a user accepts the outputs of a CDS system beyond a threshold amount - for example, the user may accept the outputs of the CDS system 90% to 100% of the time, whereas the outputs of the CDS system are known to be only 80% accurate. Under-trust can refer to a situation where a user rejects the outputs of a CDS system below a threshold amount - for example, a user may reject the outputs of the CDS system 50% of the time, whereas the outputs of the CDS system are known to be 90% accurate. Additionally or alternatively, over-trust and under-trust can relate to specific user responses to specific outputs of a CDS system. For example, over-trust can refer to a situation where a user quickly accepts (e.g., in a second or less) an output of a CDS system that is associated with a low confidence score (e.g., 50% or less). Under-trust can refer to a situation where a user quickly rejects (e.g., in a second or less) an output of a CDS system that is associated with a high confidence score (e.g., 70% or more).

Disclosed herein are systems and related methods for evaluating user interactions with CDS systems, which may be AI-based. The disclosed technology continuously monitors user interactions (e.g., to detect or evaluate user behaviors) with a CDS system to identify situations of under-trust and over-trust by a user in outputs of the CDS system. If such situations are identified, the system may take one or more corrective actions (e.g., guardrail corrections) to increase trust versus vigilance towards the CDS system. In this way the system automatically calibrates the user's trust to an appropriate trust level. An appropriate trust level may be a situation in which a user's reliance on outputs of a CDS system corresponds to accuracy of the outputs. For example, for a CDS system that is known to be 80% accurate, a user appropriately trusts the CDS system when the user accepts the outputs of the CDS system approximately 80% of the time. Appropriate trust may be defined as a range (e.g., 75% to 85% trust for a CDS system that is 80% accurate). Additionally or alternatively, appropriate trust may be specific to an interaction, such as a user decision to accept or reject an advice item generated by a CDS system.

Advantages of the disclosed technology may include, without limitation, improved (e.g., more accurate) decision making, such as for decisions related to diagnosis or treatment of medical conditions. Additionally, the disclosed technology may encourage adoption of effective CDS systems and encourage appropriate user trust in CDS systems. While example embodiments are described with reference to CDS systems, the disclosed technology can be similarly applied to other decision support systems (DSSs) and/or DSSs included in or coupled with other computing devices.

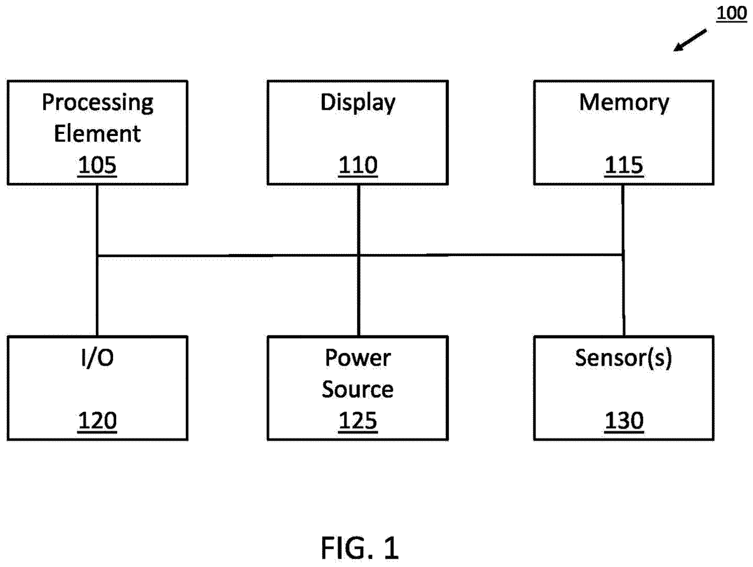

Fig. 1 is a block diagram illustrating a computing device 100 implementing a system for evaluating user interactions with a CDS system, in some implementations. For example, the computing device 100 can be one or more servers and/or user devices. In some embodiments, the computing device 100 may implement the CDS system as well as the system for evaluating user interactions with the CDS system.

The computing device 100 includes one or more processing elements 105, displays 110, memory 115, an input/output interface 120, power sources 125, and/or sensor(s) 130, each of which may be in communication either directly or indirectly.

The processing element 105 can be any type of electronic device and/or processor capable of processing, receiving, and/or transmitting instructions. For example, the processing element 105 can be a microprocessor or microcontroller. Additionally, it should be noted that select components of the system may be controlled by a first processor and other components may be controlled by a second processor, where the first and second processors may or may not be in communication with each other. The device 100 may use one or more processing elements 105 and/or may utilize processing elements included in other components. For example, a first device 100 can be a user device, while a second device 100 can be a server, and the first device 100 can use a processing element 105 of the second device 100.

The display 110 provides visual output to a user (e.g., corrective actions/guardrail corrections) and optionally may receive user input (e.g., through a touch screen interface). The display 110 may be substantially any type of electronic display, including a liquid crystal display, organic liquid crystal display, and so on. The type and arrangement of the display depends on the desired visual information to be transmitted (e.g., can be incorporated into a wearable item such as glasses, or may be a television or large display, or a screen on a mobile device).

The memory 115 stores data used by the device 100 to store instructions (e.g., software modules) for the processing element 105, and the memory 115 stores data for the system, such as medical images or other medical data, user data and user interaction data, user profiles, CDS systems and associated data, and so forth. The memory 115 may be, for example, magneto-optical storage, read-only memory, random-access memory, erasable programmable memory, flash memory, or a combination of one or more types of memory components. The memory 115 can include, for example, one or more non-transitory computer-readable media carrying instructions configured to cause the processing element 105 and/or the device 100 or other components of the system to perform operations described herein.

The I/O interface 120 provides communication to and from the various components within the device 100. The I/O interface 120 can include one or more input buttons, switches, or other input devices (e.g., keyboard, control pad, touch pad, mouse). The I/O interface 120 may further include a communication interface, such as WiFi, Ethernet, or the like, as well as other communication components, such as universal serial bus (USB) cables, or the like. In some implementations, the I/O interface 120 can be configured to receive voice inputs and/or gesture inputs. The I/O interface 120 may receive inputs from users or other systems. For example, a user may provide inputs via a keyboard or electronic medical data may be provided via Ethernet. The I/O interface may provide inputs to the appropriate components. For example, user inputs for a user interaction may be provided to the processing element 105 and/or memory 115 in relation to evaluation of a user interaction.

The power source 125 provides power to the various computing resources and/or devices. The system may include one or more power sources, and the types of power source may vary depending on the component receiving power. The power source 125 may include one or more batteries, wall outlet, cable cords (e.g., USB cord), or the like.

The sensor(s) 130 can comprise one or more cameras, microphones, touch-sensitive components, temperature sensors, motion sensors, biometric sensors, or the like. Sensor(s) 130 can capture data used to evaluate user interactions with a CDS system. For example, a sensor 130 can capture video or still images of a user to evaluate the user and/or one or more interactions of the user with a CDS system.

Components of the device 100 are illustrated only as examples, and it will be appreciated that illustrated components can be removed from and/or added to the device 100 without deviating from the teachings of the present disclosure.

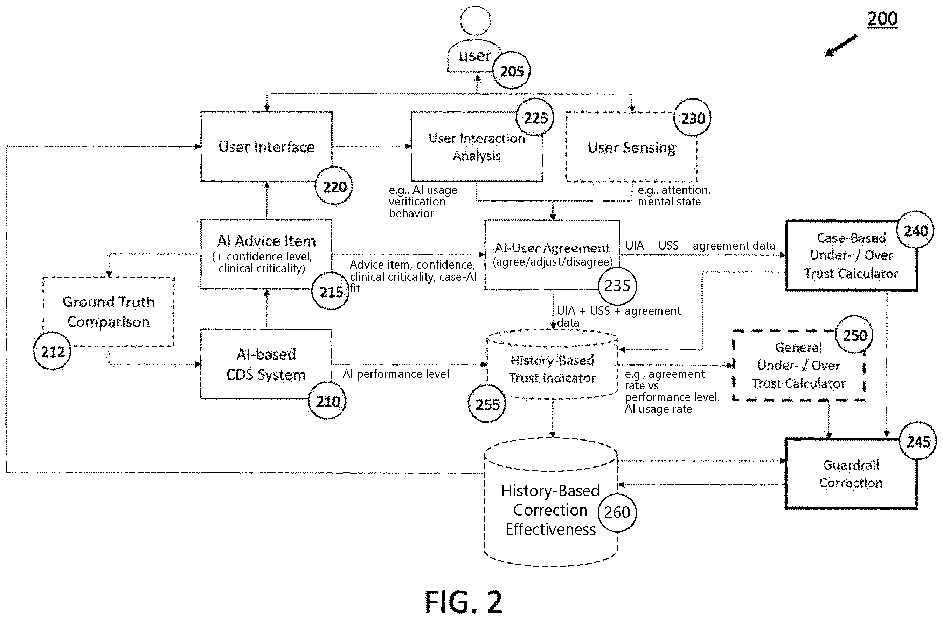

Fig. 2 is a block diagram illustrating a workflow 200 for evaluating interactions between a user 205 and an AI-based CDS system 210 in accordance with principles of the present disclosure. The workflow 200 can be performed using the computing device 100 of Fig. 1. As used herein, a "module" can refer to one or more computer executable instructions that may be part of a larger set of computer executable instructions (e.g., software program). The instructions may be executed by one or more processors, such as processing element 105. The instructions and data analyzed by the processor based on the instructions may be stored in one or more memories, such as memory 115. In some applications, the AI-based CDS system 210 may be a module (or multiple modules) in a software program that also includes one or more modules for evaluating interactions with the AI-based CDS system 210.

The workflow 200 is performed in relation to one or more interactions of the user 205 with the AI-based CDS system 210. The user 205 uses one or more medical devices or systems or computing devices that comprise or use the AI-based CDS system 210. The AI-based CDS system 210 can comprise or use one or more AI models (e.g., ML models). The AI-based CDS system 210 may have a defined accuracy, which can be expressed as a performance level (e.g., a percentage). In an example implementation, the user 205 is a clinician who uses an ultrasound imaging system to evaluate a subject, and the ultrasound imaging system uses the AI-based CDS system 210 to generate one or more outputs to the user to assist in the evaluation of the subject. In some applications, the AI-based CDS system 210 may be included in the ultrasound imaging system, but in other applications, the AI-based CDS system 210 may be included in a different computing device or system that receives data (e.g., ultrasound images) from the ultrasound imaging system for analysis. In the example implementation, the AI-based CDS system 210 can generate outputs based on received ultrasound imaging data, such as suggestions of views to capture, proposed identification of a target anatomy or other object, suggestions of settings for the ultrasound imaging system, measurements, quality metrics (e.g., for images or measurements) and so forth.

The AI-based CDS system 210 generates an output comprising an AI advice item 215. The AI advice item 215 can be, for example, a suggestion generated by the AI-based CDS system 210. Non-limiting examples of AI advice items 215 include clinical advice in medical imaging, radiology, ultrasound, radiotherapy, pathology, patient monitoring, surgery, surgery planning, general healthcare, nursing, or in any other health care domain or specialty. The AI advice item 215 is provided to the user 205 via a user interface 220 (e.g., via display 110). The user interface 220 can be provided by the AI-based CDS system 210 or an underlying system that uses the AI-based CDS system 210 (e.g., an ultrasound imaging system). The AI advice item 215 can include, for example, a confidence level associated with an output of the AI-based CDS system 210. The confidence level can be expressed as a confidence score and/or a percentage indicating a likelihood that the output is accurate. For example, the AI-based CDS system 210 can output an automatic measurement and a confidence score indicating likely accuracy of the automatic measurement (e.g., 70% accurate, 80% accurate, 90% accurate, 99% accurate). Additionally or alternatively, the AI advice item 215 can include an indication of clinical criticality (e.g., whether the AI advice item 215 relates to a life-threatening condition or decision). The indication of clinical criticality can include a score, a rating, or the like indicating relative importance of a corresponding output of the AI-based CDS system 210. A relatively high criticality score (e.g., 70% to 100%) can indicate that a corresponding output is highly important to one or more clinical decisions or processes, and a relatively low criticality score (e.g., 0% to 30%) can indicate that the corresponding output is relatively unimportant to one or more clinical decisions or processes. In some implementations, the AI advice item 215 includes a case-AI fit score, which can indicate (e.g., as a numerical score) how closely a current clinical case matches with clinical cases used for training the AI-based CDS system 210.

In various implementations, a ground truth comparison 212 is performed using the AI advice item 215. For example, the AI-based CDS system 210 can be trained by comparing the AI advice item 215 to a ground truth. The comparison can be used to determine a loss function and/or to adjust one or more weights associated with the AI-based CDS system 210. In an example implementation, the AI advice item 215 is stored until an associated ground truth is known. For example, the AI advice item 215 may include a determination of a tumor being benign or malignant. Once the AI advice item 215 is confirmed or contradicted (e.g., based on user agreement or disagreement), the result can be stored as a ground truth, and the comparison of the AI advice item 215 with the ground truth is fed back to the AI-based CDS system 210 to modify or retrain the AI-based CDS system 210.

The disclosed system includes a user interaction analysis module 225 and/or a user sensing module 230 to evaluate the user 205 and/or one or more interactions of the user with the AI-based CDS system 210. In some implementations, the user interaction analysis module 225 and/or the user sensing module 230 can continuously monitor the user and user interactions with the AI-based CDS system 210, such as interactions wherein the user 205 receives and responds to AI advice items 215 by accepting, rejecting, or modifying the AI advice items 215 (e.g., through user interface 220). The user interaction analysis module 225 can make various determinations, such as the user's 205 usage of the AI-based CDS system 210 and verification behavior associated with the AI-based CDS system 210. For example, the user interaction analysis module 225 can determine whether the user has disabled or muted the AI-based CDS system 210 and related conditions, such as whether the AI-based CDS system 210 is turned off immediately or after a period of time. The user interaction analysis module 225 can further determine user acceptance or rejection of outputs generated by the AI-based CDS system 210 and related conditions, such as a period of time before the output is accepted or rejected. Acceptance, rejection, or other interactions of the user 205 may be provided via user interface 220, which may include one or more elements of the I/O interface 120. The user interaction analysis module 225 may analyze individual interactions and multiple user interactions (e.g., to identify trends). The user interaction analysis module 225 can also detect whether a user consults other resources (e.g., clinical guides, images from previous exams of patient) before accepting or rejecting an output of the AI-based CDS system 210.

The user sensing module 230 can monitor the user 205 and make various determinations related to the user 205. The user sensing module 230 can comprise or use one or more sensors (e.g., sensor(s) 130 of Fig. 1) to monitor the user, such as a camera or a biometric sensor (e.g., to measure heartrate, galvanic skin response, neural activity). Based on captured sensor data related to the user 205, the user sensing module 230 can make determinations regarding a user's attention level or mental state. The user sensing module 230 can implement various technologies, such as computer vision, gaze detection, sentiment or emotion analysis, biometric analysis, and/or combinations thereof. In an example implementation, the user sensing module 230 monitors the user's 205 facial expressions using a camera and makes one or more determinations based on detected facial expressions. For example, a fatigued facial expression may indicate that the user's 205 attention is likely to be lacking. In these and other implementations, the user sensing module 230 can use a camera to detect the user's 205 gaze to determine, for example, whether the user 205 has seen the AI advice item 215 in the user interface 220, or to determine whether the user's 205 attention level is high or low. Additionally or alternatively, the user sensing module 230 can detect user fatigue based on one or more of pupil dilation, skin characteristics, or activity tracking. Additionally or alternatively, the user sensing module 230 can detect user tension or stress (e.g., which may indicate likelihood of under-trust or disagreement) based on one or more of skin conductance, heart rate, breathing rate, neural activity, or facial expression analysis. In these and other implementations, the user sensing module 230 can detect environmental distractions (e.g., indicating low user attention) based on one or more of sound levels, light levels, or detecting presence of people other than the user (e.g., using a camera or microphone). In some examples, the user sensing module 230 determines or estimates user cognitive load (e.g., which may indicate that the user is considering the AI advice item 215) based on one or more of pupil dilation, facial expression, or neural activity.

For a specific AI advice item 215, the disclosed technology determines AI-user agreement 235. The AI-user agreement 235 can indicate whether the user 205 agrees or disagrees with the AI advice item 215. For example, the AI-user agreement 235 can comprise a determination that the user 205 accepts the AI advice item 215 without modification, a determination that the user 205 rejects the AI advice item 215, or a determination that the user 205 accepts the AI advice item 215 after one or more adjustments. AI-user agreement 235 can be determined based on a user input or automatically (e.g., based on the user sensing module 230).

The disclosed technology includes a case-based under-/over-trust calculator 240 that makes a determination, for a particular AI advice item 215, regarding whether the user 205 exhibits under-trust, appropriate trust, or over-trust. In other words, the case-based under-/over-trust calculator 240 determines, based on the AI-user agreement 235, whether the user 205 accepts an output of the AI-based CDS system 210 too readily (e.g., an output that should have been rejected or an output that was accepted without critical evaluation) or rejects an output that should have been accepted or considered more carefully.

Over-trust and under-trust determinations made by the case-based under-/over-trust calculator 240 can be based on various factors. For example, the case-based under-/over-trust calculator 240 can determine a time between generation of the output by the AI-based CDS system 210 and a user response rejecting or accepting the output to infer whether the user responded without adequately considering the output (e.g., before expiration of a threshold time period). Additionally or alternatively, the case-based under-/over-trust calculator 240 can receive an output from the user interaction analysis module 225 and/or the user sensing module 230 to determine, for example, whether the user's 205 attention level is high or low, whether the user 205 saw the AI advice item 215, how long the user 205 looked at the AI advice item 215, and so forth. Additionally or alternatively, the case-based under-/over-trust calculator 240 can consider characteristics of an AI advice item 215 to determine over-trust or under-trust. For example, the case-based under-/over-trust calculator 240 can receive a confidence level (e.g., confidence score) and/or a criticality level for the AI advice item 215. User acceptance of an output having a low confidence score may indicate over-trust, whereas user rejection of an output having a high confidence score may indicate under-trust.

In an example implementation, the case-based under-/over-trust calculator 240 receives data from or including the AI advice item 215, the user interaction analysis module 225, the user sensing module 230 and/or the AI-user agreement 235, and the case-based under-/over-trust calculator 240 determines a case-specific trust value representing a position on a trust scale for the AI advice item 215. The trust value can be expressed, for example, on a trinary scale of -1 (under-trust), to 0 (appropriate trust), to +1 (over-trust); on a Likert scale of -7 (severe under-trust) to +7 (severe over-trust) (where 0 is appropriate trust); on a continuous scale of -100 (severe under-trust) to +100 (severe over-trust) (where 0 is appropriate trust); or combinations thereof. The trust value can be determined, for example, using an if-this-then-that or other type of rule-based system (e.g., using predetermined criteria), using a formula (e.g., based on defined variables and corresponding values), and/or using a trained deep learning algorithm or other AI model.

An example of a rule-based system for producing a trust value, which may be implemented by the case-based over-/under-trust calculator 240, is illustrated in Table 1 below. In this example, the trust value is expressed on a scale of -100% (under-trust) to +100% (over-trust). Factors considered by the example rule-based system include AI confidence level (AICL), AI-User Agreement (LTA), and User Interaction Analysis comprising Decision Time (DT), Verification Time (VT), and Verification Amount (VA). UA is expressed as a number between 0 and 100%, where 0 indicates a rejected advice item and 100% indicates an advice item accepted without modification. When a user adjusts an advice item before accepting, agreement will be lower, such as 50% when half of the advice content is accepted and half is adjusted. DT is expressed as length of time in seconds between the advice item appearing on the display and the user deciding whether to accept it. VT is expressed as length of time in seconds the user spends with AI verification sources (e.g. explanations, manual, source data) opened. Trust values correspond to respective guardrail corrections (e.g., corrective actions), which may be associated with weights (e.g., lightweight or strong).

| **TABLE 1** | | | | | |
|---|---|---|---|---|---|
| **AICL (confidence)** | **UA (acceptance)** | **Verification Time** | **Decision Time** | **Trust Value** | **Guardrail Correction** |
| 95% | 100% | 3-9 | 3-9 | 0 | No |
| 75% | 0% | 3-9 | 3-9 | 0 | No |
| 95% | 0% | 10+ | 10+ | -10% | No |
| 75% | 100% | 10+ | 10+ | 10% | No |
| 95% | 100% | <3 | <3 | 30% | Yes - lightweight |
| 75% | 0% | <3 | <3 | -30% | Yes - lightweight |
| 95% | 50% | 3-9 | 3-9 | -10% | No |
| 75% | 50% | 3-9 | 3-9 | 0 | No |
| 95% | 0% | <3 | <3 | -100% | Yes - strong |
| 75% | 100% | <3 | <3 | 100% | Yes - strong |

Based at least in part on a determination of the case-based under-/over-trust calculator 240, the disclosed technology may generate a guardrail correction 245 (e.g., a corrective action), such as in response to a determination of under-trust or over-trust. For example, the disclosed technology may generate and provide to the user 205 (e.g., via the user interface 220) a prompt or notification configured to correct for under-trust or over-trust. The prompt or notification may encourage the user 205 to revisit a decision to accept or reject the AI advice item 215. In these and other implementations, the prompt or notification may request or require confirmation from the user 205, such as a confirmation that the user rejects an AI advice item 215 (e.g., particularly where the AI advice item 215 is associated with a high confidence) or that the user accepts an AI advice item 215 (e.g., particularly where the AI advice item 215 is associated with a low confidence). Guardrail corrections 245 may comprise visual, auditory, and/or tactile features, such as text, flashing lights, buzzing or beeping sounds, narrations, vibrations, and so forth.

Determining whether to generate a guardrail correction 245 can be based on one or more thresholds. For example, a guardrail correction 245 can be generated when under-trust or over-trust meets or exceeds a threshold (e.g., -30% or +30% on a scale of -100% to +100%). The threshold can be modified, such as based on different use cases or user preferences. For example, the threshold for generating the guardrail correction 245 can be lower when the AI advice item 215 includes an indication of high criticality - that is, a guardrail correction 245 can be triggered at a lower threshold when the AI advice item 215 relates to a critical aspect of an evaluation or determination related to a subject. In some implementations, guardrail corrections 245 can have different weights, and multiple thresholds can be used to determine a weight of the guardrail correction 245. A lightweight guardrail correction 245 can be, for example, a simple visual prompt that does not require a response or input from the user 205, whereas a strong guardrail correction 245 is more noticeable, such as by including an auditory component or a flashing visual indicator or requiring a user response or input (e.g., a confirmation from the user 205). In some examples, a lightweight guardrail correction 245 can be generated when under-trust or over-trust meets or exceeds a first threshold (e.g., -30% or +30% on a scale of -100% to +100%), and a strong guardrail correction 245 can be generated when under-trust or over-trust meets or exceeds a second threshold (e.g., -80% or +80% on a scale of -100% to +100%). While an example is described using two thresholds, it will be appreciated that any number of thresholds and corresponding weights for guardrail corrections 245 can be used, such as multiple thresholds corresponding with escalating guardrail corrections 245 as over-trust or under-trust increases.

In some implementations, the guardrail correction 245 can be a modification to one or more outputs of the AI-based CDS system 210. For example, where the user 205 exhibits under-trust by ignoring an AI advice item 215, the guardrail correction 245 may cause subsequent AI advice items 215 to be more noticeable, such as by adding an auditory or tactile component in addition to visual component, increasing a font size, displaying a pop-up window, displaying a flashing notification, or the like. Where the user 205 exhibits over-trust, the guardrail correction may cause subsequent AI advice items 215 to be less noticeable, such as by decreasing font size, making a notification smaller, or removing components (e.g., auditory or tactile components) of subsequent AI advice items 215.

In some implementations, the disclosed technology includes a general under-/over-trust calculator 250 in addition to the case-based under-/over-trust calculator 240, and the guardrail correction 245 is further based on a determination of the general under-/over-trust calculator 250. For example, whereas the case-based under-/over-trust calculator 240 may consider characteristics associated with a specific AI advice item 215, the general under-/over-trust calculator 250 may consider broader characteristics, such as characteristics of the AI-based CDS system 210 itself (e.g., overall accuracy or other performance characteristics) and/or characteristics of the user 205 (e.g., user history, user trust in AI tools, agreement rate, user fatigue, user experience level, user response to previous guardrail corrections 245). In some examples, trust determinations generated by the case-based under-/over-trust calculator 240 and the general over-/under-trust indicator can be combined (e.g., as a combined and/or weighted trust score), and one or more thresholds can be applied to the combined trust determination to determine whether to generate a guardrail correction 245 and/or a weight of a guardrail correction 245.

Table 2 below illustrates non-limiting examples of under-trust, over-trust, and appropriate trust, corresponding characteristics of a user interaction, and corresponding guardrail corrections 245 generated by the disclosed system.

| **TABLE 2** | | |
|---|---|---|
| **State** | **Interaction Characteristics** | **Guardrail Correction** |
| Under-trust | -Fast rejection (e.g., < 1 second) | -Visual output to encourage greater trust in the CDS system |
| | -High confidence score | |
| | -User history of low trust | |
| | -Low user attention/ignoring outputs | |
| Appropriate trust | -User response after appropriate consideration period (e.g., 3-9 seconds) | -Visual output confirming appropriate trust in the CDS system |
| | -History of user agreement correlated to accuracy of the CDS system | -Alternatively, no guardrail correction generated |
| | -Appropriate/high user attention and/or lack of user fatigue | |
| Over-trust | -Fast acceptance (e.g., < 1 second) | -Visual output to encourage user evaluation of CDS system outputs before acceptance |
| | -Low confidence score | |
| | -User history of high trust (e.g., exceeding accuracy of CDS system) | |
| | -User fatigue or low user attention | |

In some implementations, the general under-/over-trust calculator 250 uses data provided by a history-based trust indicator 255. For example, the history-based trust indicator 255 can be a database or other storage that stores data related to user history for one or more users 205. The history-based trust indicator 255 can be used to track the user's 205 trust in the AI-based CDS system 210 over time. For example, the user's 205 rate of agreement with outputs of the AI-based CDS system 210 can be compared to a performance level (e.g., an accuracy) of the AI-based CDS system 210, and the comparison can be used as an indication of likely over-trust or under-trust. Additionally or alternatively, the comparison can be used to evaluate whether the user's 205 general trust level has increased or decreased over time and/or whether the user's 205 trust level is appropriate (e.g., whether a general trust level corresponds to accuracy of the AI-based CDS system 210). In various implementations, the history-based trust indicator 255 can comprise or be included in a user profile of the user 205, which can provide or store various user characteristics, such as user identifiers, user preferences, user history, user permissions, and so forth.

In various implementations, the disclosed technology can determine history-based correction effectiveness 260. For example, an effectiveness of a generated guardrail correction 245 can be determined based on how the user 205 responds to the guardrail correction 245. A guardrail correction 245 can be considered relatively effective, for example, where the user 205 changes a decision to accept or reject an output of the AI-based CDS system 210 in response to the guardrail correction 245. A guardrail correction 245 can be considered relatively ineffective, for example, where the user 205 ignores the guardrail correction 245.

One or more operations and/or modules included in the workflow 200 can be implemented using an AI model (e.g., a ML model). For example, an AI model can be used to implement the user interaction analysis module 225, the user sensing module 230, and or to generate the guardrail correction 245. While example modules are discussed in relation to the workflow 200, a person skilled in the art will appreciate that more or fewer modules may be used, and one or more modules can be removed or combined while maintaining a similar functionality. Additionally, operations of the workflow 200 can be removed, added, combined, or repeated while maintaining a similar functionality, and one or more operations of the workflow 200 can be performed in parallel.

As used herein, a "model" can refer to a construct that is trained using training data to make predictions or provide probabilities for new data items, whether or not the new data items were included in the training data. For example, training data for supervised learning can include items with various parameters and an assigned classification. A new data item can have parameters that a model can use to assign a classification to the new data item. As another example, a model can be a probability distribution resulting from the analysis of training data, such as a likelihood of an n-gram occurring in a given language based on an analysis of a large corpus from that language. Examples of models and/or associated techniques include, without limitation: neural networks, support vector machines, decision trees, Parzen windows, Bayes, clustering, reinforcement learning, probability distributions, decision trees, decision tree forests, and others. Models can be configured for various situations, data types, sources, and output formats. A model trained or provided by the disclosed system can include a neural network with multiple input nodes that receive training datasets. The input nodes can correspond to functions that receive the input and produce results. These results can be provided to one or more levels of intermediate nodes that each produce further results based on a combination of lower-level node results. A weighting factor can be applied to the output of each node before the result is passed to the next layer node. At a final layer, ("the output layer,") one or more nodes can produce a value classifying the input that, once the model is trained, can be used to evaluate new data (e.g., to evaluate a user interaction or a user, to evaluate user trust, or to generate a guardrail correction). In some implementations, such neural networks, known as deep neural networks, can have multiple layers of intermediate nodes with different configurations, can be a combination of models that receive different parts of the input and/or input from other parts of the deep neural network, or are convolutions-partially using output from previous iterations of applying the model as further input to produce results for the current input.

A model can be trained with supervised learning (e.g., self-supervised). Testing data can then be provided to the model to assess accuracy. Testing data can be, for example, a portion of the entire dataset (e.g., 10%) held back to use for evaluation of the model. Output from the model can be compared to the desired or expected output for the training data and, based on the comparison, the model can be modified, such as by changing weights between nodes of the neural network and/or parameters of the functions used at each node in the neural network (e.g., applying a loss function). Based on the results of the model evaluation, and after applying the described modifications, the model can then be retrained to evaluate new data.

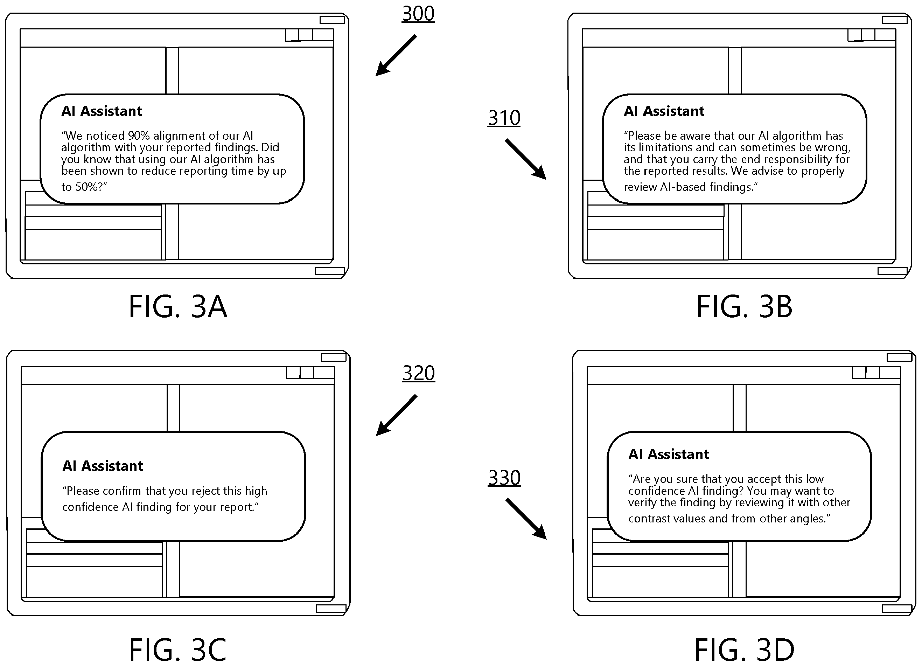

Fig. 3A is a display diagram illustrating an output 300 of a system for evaluating user interactions with a CDS system (e.g., the AI-based CDS system 210 of Fig. 2) arranged in accordance with principles of the present disclosure. The output 300 can be a guardrail correction 245 generated by a system implementing the workflow 200 of Fig. 2. In some implementations, the outputs 300 may be provided on a display, such as display 110.

In an example implementation, the CDS system generates various outputs related to user interactions with a device or system, such as a medical imaging device. As discussed herein, the outputs can include, for example, automatic measurements, identification of a target anatomy or other object, quality assessments, suggested settings, and so forth. A user (e.g., 205 of Fig. 2) takes various actions in response to the outputs of the CDS system, such as actions to accept, reject, ignore, or modify the outputs of the CDS system. The disclosed technology may continuously monitor the user and/or user interactions to evaluate user agreement or disagreement with the CDS system and other characteristics of user interactions, and the disclosed technology may generate and display the output 300 based on this evaluation.

The output 300 provides information to the user regarding alignment between the user's findings, which may be manually entered, and outputs of the CDS system (e.g., "We noticed 90% alignment of our AI algorithm with your reported findings."). The output 300 may be displayed responsive to detecting a trend of rejecting high-confidence findings of the CDS system despite substantial agreement between the findings and the user's findings. The output 300 may be configured to encourage appropriate trust in the CDS system, such as by reminding the user of the accuracy of the CDS system and informing the user of the substantial alignment between the user's findings and the findings of the CDS system.

Fig. 3B is a display diagram illustrating another output 310 of the system for evaluating user interactions with the CDS system, as illustrated with reference to Fig. 3A. In this example, the output 310 includes an indication that outputs of the CDS system should be properly reviewed before being accepted by the user (e.g., "our AI algorithm has its limitations" and "[w]e advise to properly review AI-based findings"). The output 310 may be generated and displayed, for example, where a user exhibits over-trust in the CDS system - that is, where the user accepts one or more outputs of the CDS system too quickly, with a low confidence score, beyond a threshold amount or an acceptable range, or the like. The output 310 may be generated responsive to one or more of the following characteristics: user fatigue or low user attention (e.g., based on gaze detection and/or computer vision using a camera), fast acceptance of CDS system outputs (e.g., < 1 second), acceptance of outputs of CDS system having a low confidence score, user history of over-trust.

Fig. 3C is a display diagram illustrating another output 320 of the system for evaluating user interactions with the CDS system, as illustrated with reference to Figs. 3A & 3B. In this example, the output 320 includes a request or requirement for confirmation that an output of the CDS system has been rejected despite high confidence that the output of the CDS system is accurate. For example, the CDS system may generate an output and a corresponding confidence score that is relatively high (e.g., 80%, 90%, 95%), which is nonetheless rejected by the user. In this situation, the disclosed technology may display the output 320 to encourage greater trust in the CDS system by informing the user of the high confidence in the output and requiring confirmation of the user's decision to nonetheless reject the output. The output 320 may be generated responsive to one or more of the following characteristics: ignoring CDS system outputs (e.g., based on gaze detection and/or computer vision using a camera), fast rejection of CDS system outputs (e.g., < 1 second), rejection of outputs of CDS system having a high confidence score, user history of under-trust.

Fig. 3D is a display diagram illustrating another output 330 of the system for evaluating user interactions with the CDS system, as illustrated with reference to Figs. 3A-3C. In this example, the output 330 includes a request or requirement for confirmation that an output of the CDS system has been accepted despite low confidence that the output of the CDS system is accurate. For example, the CDS system may generate an output and a corresponding confidence score that is relatively low (e.g., 5%, 10%, 20%, 30%), which is nonetheless accepted by the user. In this situation, the disclosed technology may display the output 330 to encourage the user to reconsider the decision to accept the low-confidence output of the CDS system. The output 330 may be generated based on similar characteristics that may result in the output 310 of Fig. 3B.

In the examples of Figs. 3A-3D, the outputs 300-330 comprise text outputs or prompts generated using an AI chatbot (e.g., a virtual chatbot provided by the disclosed system). In these and other implementations, the disclosed system may generate additional or alternative outputs, which may include other visual, auditory, and/or tactile outputs. In some implementations, the outputs may change over time and/or responsive to changed conditions. For example, if repeated text outputs are unsuccessful in changing a user's trust level in the CDS system, then the disclosed system may generate outputs with increasing frequency, with more noticeable visual features or characteristics (e.g., flashing visual indicators, larger text, brighter colors), with auditory outputs (e.g., a buzzing or beeping sound, a speech notification), or with tactile outputs (e.g., vibrating a portion of a device in contact with a user).

While example outputs 300-330 are shown, additional or alternative outputs can be provide while maintaining a similar functionality. The outputs 300-330 can be generated automatically by the disclosed system and/or the outputs 300-330 can be selected by the disclosed system from a set of predetermined outputs

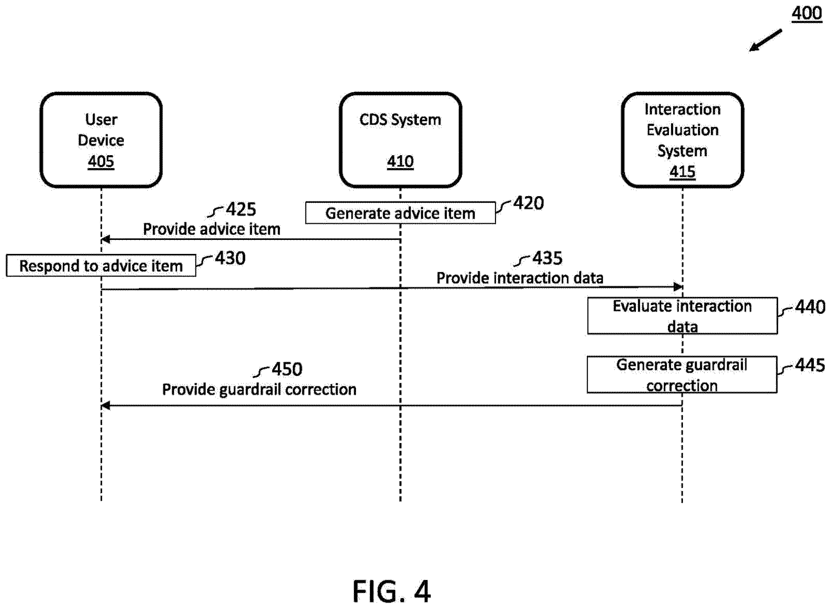

Fig. 4 is a sequence diagram illustrating a process 400 for evaluating user interactions with a CDS system in accordance with principles of the present disclosure. The process 400 can be performed using one or more modules and/or operations described with reference to the workflow 200 of Fig. 2 to generate guardrail corrections 245 (e.g., outputs 300-330 of Figs. 3A-3D). In some embodiments, the process 400 may be implemented by a computing device, such as computing device 100 of Fig. 1. The process 400 is performed using a user device 405, a CDS system 410, and/or the disclosed interaction evaluation system 415, which may be implemented using the same computing device and/or using multiple computing devices.

At operation 420, the CDS system 410 generates an advice item, which may be an AI advice item 215 as in Fig. 2. The CDS system 410 may be the AI-based CDS system 210 of Fig. 2. The advice item generated at operation 420 may be an automated output of the CDS system 410 generated in conjunction with a user's use of a medical device or system or the user's analysis of data received from the medical device or system (e.g.., a radiologist reviewing images acquired by a sonographer). For example, the advice item may be an automated identification of a target anatomy in an ultrasound image.

At operation 425, the CDS system 410 provides the advice item generated at operation 420 to a user via a user device 405. For example, the advice item can be displayed via a graphical user interface. In an example implementation, providing the advice item comprises displaying an indication of an identified target anatomy in an ultrasound image, such as a bounding box or other shape.

At operation 430, a response to the advice item is received via the user device 405. The response may be a user response to accept, reject, ignore, or modify the advice item. For example, a user of an ultrasound imaging system may disregard an identified target anatomy and instead manually place a bounding box to indicate the location of the target anatomy.

At operation 435, interaction data related to the user's response to the advice item is provided to the interaction evaluation system 415. The interaction data can comprise the generated advice item and the user's response to accept, reject, ignore, or modify the advice item. Additionally or alternatively, the interaction data can include a duration of time before the user responded to the advice item. Other examples of interaction data include sensor data associated with a user, such as video or still images of a user that can be evaluated to determine user mental state (e.g., attentiveness, fatigue, gaze) or other user state information, such as using gaze detection or computer vision. Sensor data can additionally or alternatively include user biometric data. Interaction data can further include user history data and/or user profile data (e.g., user history of over-trust, under-trust, or appropriate trust), accuracy of the CDS system 410, confidence associated with the advice item, or the like.

At operation 440, the interaction evaluation system 415 evaluates the interaction data to determine one or more characteristics and/or determine user over-trust, under-trust, or appropriate trust in the advice item. For example, the interaction evaluation system 415 can evaluate the interaction data to determine characteristics and associated states illustrated above with reference to Table 2. Evaluating the interaction data may be based at least in part on accuracy of the CDS system 410. Evaluating the interaction data can be based on a user characteristic, such as a user profile or a history of user interactions with the CDS system 410 or a different CDS system. In some implementations, a confidence score associated with the advice item is generated, and the evaluation of the interaction data is based at least in part on the confidence score.

At operation 445, the interaction evaluation system 415 generates a guardrail correction (corrective action) based on the evaluation of the interaction data at operation 440. For example, the guardrail correction can be a visual prompt or notification to encourage appropriate trust, such as the outputs 300-330 of Figs. 3A-3C and/or guardrail corrections described with reference to Table 2. As described herein, the guardrail correction generated at operation 445 may be in response to determining over-trust, under-trust, or appropriate trust. The guardrail correction can be visual, auditory, and/or tactile.

At operation 450, the interaction evaluation system 415 provides the guardrail correction to the user via the user device 405. For example, a visual component of the guardrail correction is displayed via a graphical user interface, an auditory component is output via a speaker or other audio device, and a tactile component is output via a tactile output device (e.g., an electric motor).

In some implementations, the interaction evaluation system 415 evaluates effectiveness of one or more guardrail corrections, such as by evaluating whether a user modifies a decision to accept or reject the advice item generated at operation 420 in response to the guardrail correction.

In some implementations, the interaction evaluation system 415 comprises or uses an AI model. In these and other implementations, the AI model can be trained using a training dataset comprising user interaction data, corresponding trust determinations, and corresponding guardrail corrections. For example, the AI model can be trained to determine user interactions that correspond to under-trust, over-trust, and appropriate trust, and the AI model can be trained to generate or select appropriate guardrail corrections based on determining under-trust, over-trust, or appropriate trust.

More or fewer operations can be included in the process 400 without deviating from the teachings of the present disclosure, and one or operations can be repeated. In some implementations, one or more operations of the process 400 are performed in parallel.

The systems and related methods disclosed herein may provide for more accurate and efficient use of CDS systems, such as AI-based CDS systems. For example, the disclosed technology encourages appropriate trust of CDS systems - that is, user trust that generally corresponds to the level of accuracy of a CDS system, rather than over-trust or under-trust of the CDS system. Additionally, the disclosed technology may encourage adoption of CDS systems. Moreover, the disclosed technology provides for real-time evaluation of user trust associated with CDS systems and real-time corrective actions based on user trust.

In various examples where components, systems and/or methods are implemented using a programmable device, such as a computer-based system or programmable logic, it should be appreciated that the above-described systems and methods can be implemented using any of various known or later developed programming languages, such as "Python", "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general-purpose processing circuits which are programmed responsive to executable instructions to perform the functions described herein.

Of course, it is to be appreciated that any one of the examples or processes described herein may be combined with one or more other examples and/or processes or be separated and/or performed amongst separate devices or device portions in accordance with the present systems, devices and methods.

As used herein, the terms "about" and "approximately" modifying, for example, a percentage of accuracy, a confidence score, and ranges thereof, employed in describing the embodiments of the disclosure, refers to variation in the numerical quantity that can occur, for example, through statistical variation due to training data set size; through image quality; through variations in noise; through variations in models used; and like proximate considerations. In some instances, the terms "about" and "approximately" include values up to and including 10% less than and 10% greater than the recited value.

Finally, the above-discussion is intended to be merely illustrative of the present systems and methods and should not be construed as limiting the appended claims to any particular example or group of examples. Thus, while the present system has been described in particular detail with reference to exemplary examples, it should also be appreciated that numerous modifications and alternative examples may be devised by those having ordinary skill in the art without departing from the broader and intended spirit and scope of the present systems and methods as set forth in the claims that follow. Accordingly, the specification and drawings are to be regarded in an illustrative manner and are not intended to limit the scope of the appended claims.

## Claims

1. A computer-implemented method (400) for evaluating user interactions with a clinical decision support (CDS) system, the method comprising:
receiving an output of the CDS system based on data acquired via a medical device (425);
accessing user interaction data associated with the output of the CDS system (435),
wherein the user interaction data comprises at least one action responsive to the output of the CDS system, wherein the at least one action indicates user agreement or disagreement with the output of the CDS system, and wherein the at least one action indicating user agreement or disagreement comprises a user input provided via a user interface (220) of the CDS system;
evaluating the user interaction data based at least in part on an accuracy of the CDS system (440); and
generating a corrective action based on the evaluating of the user interaction data (445) and providing the corrective action via the user interface (220).

2. The computer-implemented method of claim 1, wherein evaluating the user interaction data based at least in part on the accuracy of the CDS system includes determining a user trust level associated with the output of the CDS system, a user trust level associated with the CDS system, or both.

3. The computer-implemented method of claim 2, wherein the user trust level associated with the CDS system is based on at least one user characteristic, and wherein the at least one user characteristic includes a user profile or a history of user interactions with the CDS system or a different CDS system.

4. The computer-implemented method of claim 1, wherein the corrective action comprises a modification of a visual characteristic, a prompt or notification provided via the user interface (220), an auditory component, a tactile component, or combinations thereof.

5. The computer-implemented method of claim 1, further comprising:
determining a confidence score associated with the output of the CDS system, wherein the user interaction data is evaluated based at least in part on the confidence score.

6. The computer-implemented method of claim 2, further comprising:
comparing the user trust level to a threshold value, wherein the threshold value is based on an appropriate trust level corresponding to the accuracy of the CDS system, and wherein the corrective action is based on the comparing of the user trust level to the threshold value.

7. The computer-implemented method of claim 1, wherein the user interaction data is evaluated using an artificial intelligence (AI) model trained using a training dataset comprising different user interaction data, corresponding user trust data, and corresponding guardrail corrections.

8. The computer-implemented method of claim 1, further comprising:
determining an effectiveness of the corrective action based at least in part on a user input responsive to the corrective action or different user interaction data of a different user interaction.

9. A non-transitory computer-readable medium (115) carrying instructions that, when executed by a processor (105), cause the processor (105) to perform operations for evaluating user interactions with a decision support system (DSS), the operations comprising:
receiving an output of the DSS based on data acquired via a computing device (425);
accessing user interaction data associated with the output of the DSS (435), wherein the user interaction data comprises at least one action responsive to the output of the DSS, wherein the at least one action indicates user agreement or disagreement with the output of the DSS, and wherein the at least one action indicating user agreement or disagreement comprises a user input provided via a user interface (220) of the DSS;
evaluating the user interaction data based at least in part on an accuracy of the DSS (440); and
generating a corrective action based on the evaluating of the user interaction data (445) and providing the corrective action via the user interface (220).

10. The non-transitory computer-readable medium of claim 9, wherein evaluating the user interaction data based at least in part on the accuracy of the DSS includes determining a user trust level associated with the output of the DSS, a user trust level associated with the DSS, or both.

11. The non-transitory computer-readable medium of claim 10, wherein the user trust level associated with the DSS is based on at least one user characteristic, and wherein the at least one user characteristic includes a user profile or a history of user interactions with the DSS or a different DSS.

12. The non-transitory computer-readable medium of claim 9, wherein the corrective action comprises a modification of a visual characteristic, a prompt or notification provided via the user interface (220), an auditory component, a tactile component, or combinations thereof.

13. The non-transitory computer-readable medium of claim 9, further comprising:
determining a confidence score associated with the output of the DSS, wherein the user interaction data is evaluated based at least in part on the confidence score.

14. The non-transitory computer-readable medium of claim 9, wherein the user interaction data is evaluated using an artificial intelligence (AI) model trained using a training dataset comprising different user interaction data, corresponding user trust data, and corresponding guardrail corrections.

15. A system for evaluating user interactions with a clinical decision support (CDS) system (210), the system comprising one or more processors configure to:
receive an output of the CDS system based on data acquired via a medical device;
access user interaction data associated with the output of the CDS system , wherein the user interaction data comprises at least one action responsive to the output of the CDS system, wherein the at least one action indicates user agreement or disagreement with the output of the CDS system, and wherein the at least one action indicating user agreement or disagreement comprises a user input provided via a user interface (220) of the CDS system;
evaluate the user interaction data based at least in part on an accuracy of the CDS system; and
generate a corrective action based on the evaluating of the user interaction data and
provide the corrective action via the user interface (220).
